# EUROPEAN PATENT APPLICATION

(11) **EP 3 241 544 A1**
(43) Date of publication of application: **08.11.2017**
(21) Application number: 16397513.9
(22) Date of filing: 06.05.2016
(51) Int. Cl.: A61K 9/00

(54) **CELLULOSE CARTRIDGE**

(71) Applicant: Vetcare Oy, 04601 Mäntsälä (FI)
(72) Inventor: NISKANEN, Elina, 90420 Oulu (FI); KARJALAINEN, Jaana, 90420 Oulu (FI); UUSITALO, Jouko, 90420 Oulu (FI)
(74) Representative: Seppo Laine Oy

(57) **Abstract**

According to an example aspect of the present invention, there is provided a cartridge for the delivery of medicine to mammals in veterinary applications comprising a nonwoven cellulose tube having a closed end and an open end, wherein at least one cross-sectional area of the closed end is axially symmetric in relation to the longitudinal axis of the tube and the open end is plugged e.g. by folding or crushing the open end, or by inserting a cellulose cap, a disintegrating cap or a soluble cap.

## Description

### FIELD

The present invention relates to a cellulose cartridge for delivery of a substance or substances to mammals. In particular the invention relates to a cartridge suitable for use in veterinary applications, such as for the delivery of supplements to mammals. The present invention further relates to a method of preparing a cartridge. Further, the present invention relates to the use of a cartridge in veterinary applications, such as medicine.

### BACKGROUND

To prevent and cure diseases and conditions animals must be individually administered with drugs, supplements and other substances. Many of these substances are administered orally in various physical forms.

Oral administration presents a number of challenges. Many substances are unpleasant in taste and although many masking substances have been developed these have been found not entirely effective and in the main, animals, e.g. ruminants, working animals and sporting animals, find oral substances unpleasant in flavour and prefer not to ingest them.

Consequently, at least a part of the oral dosage can be ejected from the animal's mouth and intestinal system through e.g. drooling and a therapeutic dose is not achieved. The animal can also resist administration so successfully that no substance enters the mouth and intestinal system. As many of the substances taste unpleasant, simple mixing in fodder or in drinking water is not a satisfactory means of delivery as animals may refuse to ingest and consequently an adequate or therapeutic amount of the substance fails to enter the organism. A result is that symptoms of the condition or disease under which the animal labours are not alleviated and the condition or disease is not cured.

Various solutions have been developed with the aim of better controlling oral dosing. For example, in ruminants, sporting animals and working animals, solutions rely mainly on administering substances to deeper parts of animals' mouths, bypassing tongue and teeth.

Liquids can, for example, be administered *via* tubing as an elongated nozzle on a bottle or other container. There are still, however, risks associated with force feeding liquids, *e.g.* the animal can still reject the liquid or the liquid can be aspirated into the lungs of the animal.

Solids can be administered e.g. in the form of boluses using a bolus applicator or gun of a size suitable for the bolus in question. The applicator bypasses the tongue and teeth of the animal. One such bolus is disclosed in US Patent 5,395,622, which describes a preparation for the prevention or treatment of hypocalcemia in ruminants prepared by forming a mixture of calcium chloride and calcium sulphate, adding water to the mixture, introducing the mixture into moulds and heating the moulds so that the mixture solidifies. Calcium supplements, such as BoviKalc and QuikCal, trace element and vitamin supplements, such as smAll-Trace, and antibacterials, such as Sustain III Cattle are administered in bolus form. Oftentimes, boluses are manufactured using xenobiotic substances in order to bind the components of the bolus or to adjust release time of the components of the bolus. There is, however, only a very limited number of substances readily available as solid boluses, leaving veterinary practitioners and animal owners struggling to provide correct doses, especially with the administration of powders, capsules, pills, tablets and other solid forms.

WO 2006/072252 describes a powdered preparation containing a calcium source enclosed in a substantially water soluble, dispersible or disintegrable casing mainly composed of a cellulose material closed at both ends.

As mentioned above, solid form drugs and dietary supplements are typically mixed with dry or wet feed and offered to the animal as such. Solids can also be dissolved in or mixed with water and provided to the animal *e.g. via* a nozzled bottle or feeding tube. An example of such a drug is ketoprofen. Although it is available in injectable form, animals are often prescribed powders for mixing with feed and/or water.

Monitoring devices, medical devices and magnets must be shaped such that easy passage through the mouth and oesophagus is allowed. The Ecow farmBolus for pH and temperature measurements e.g. is rounded at each end to allow for easy passage.

### SUMMARY OF THE INVENTION

It is an aim of the present invention to overcome at least some of the above-mentioned disadvantages and to provide a cartridge suitable for use medicine, e.g. in veterinary applications, such as for the delivery of supplements to mammals. It is a still further aim of the present invention to provide a method of producing a cartridge. A further aim of the invention is to provide a kit comprising a cartridge and an applicator or delivery gun.

The invention is defined by the features of the independent claims. Some specific embodiments are defined in the dependent claims.

According to a first aspect of the present invention, there is provided a cartridge suitable for delivery of medicine to mammals in veterinary applications comprising a non-woven cellulose tube having a closed end and an open end, , wherein at least one cross-sectional area of the closed end is axially symmetric in relation to the longitudinal axis of the tube and the open end is plugged *e.g.* by folding or crushing the open end, or by inserting a cellulose cap, a disintegrating cap or a soluble cap.

According to a second aspect of the present invention, there is provided a cartridge suitable for delivery of medicine to mammals in veterinary applications comprising a non-woven cellulose tube having a closed end and an open end, wherein at least one cross-sectional area of the closed end is axially symmetric in relation to the longitudinal axis of the tube for use in medicine.

According to a third aspect of the present invention, there is provided a method of producing a cellulose cartridge for the delivery of medicine to mammals comprising moulding a feed grade cellulose-water paste on a mould to produce a single homogenous cartridge, drying the cartridge.

According to a fourth aspect of the present invention, there is provided a kit comprising a cartridge and a delivery applicator.

### BRIEF DESCRIPTION OF THE DRAWINGS

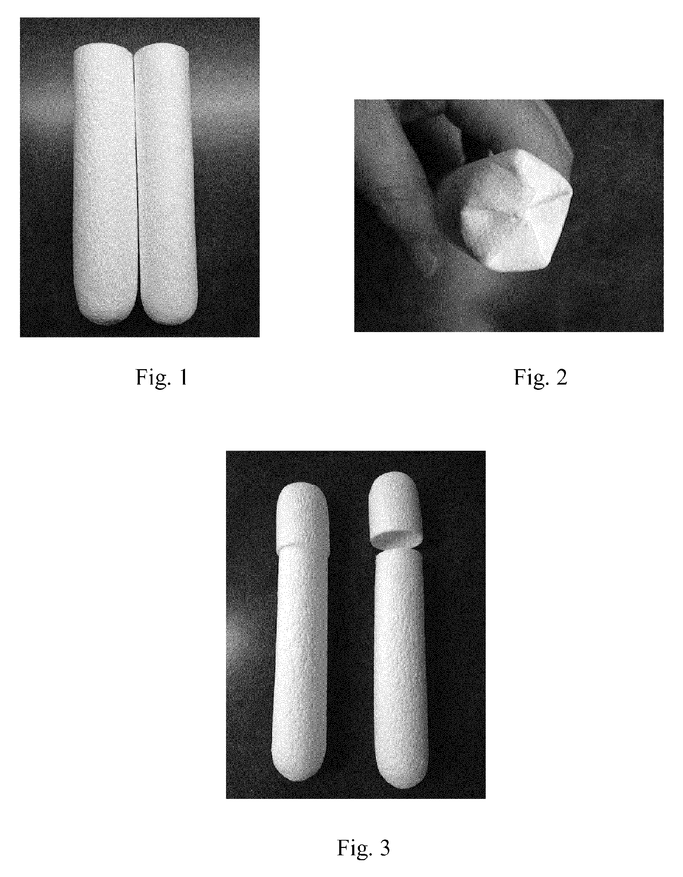

FIGURE 1 illustrates a cartridge for the delivery of medicine to mammals in accordance with at least some embodiments of the present invention;
FIGURE 2 illustrates a cartridge according to at least some embodiments of the invention, which cartridge's open end has been closed by folding.
FIGURE 3 illustrates a cartridge closed with a rounded cap as well as a cartridge and closing cap.

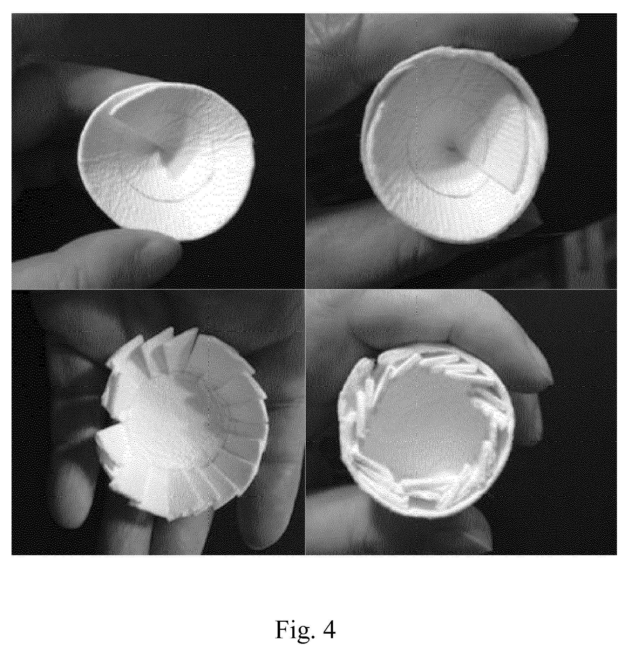

FIGURE 4 illustrates various closing caps suitable for closing cartridges in accordance with at least some embodiments of the present invention.

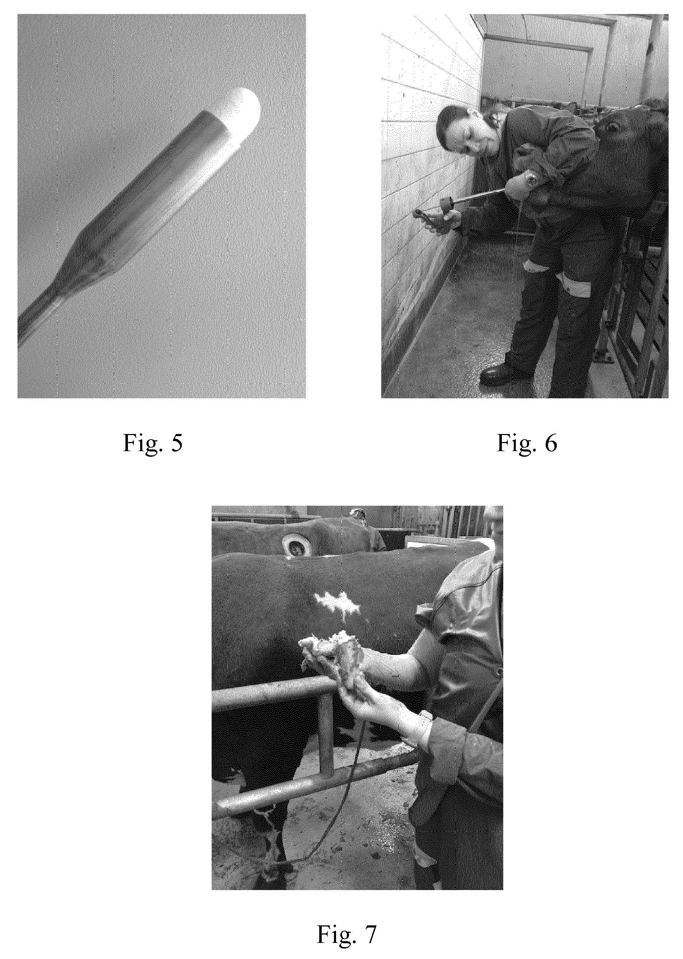

FIGURE 5 shows a cartridge in an applicator.
FIGURE 6 shows application of the cartridge to a cow.
FIGURE 7 shows a partly disintegrated cartridge caught in and removed from the rumen of the cow one minute after application.

### EMBODIMENTS

### DEFINITIONS

By veterinary applications is meant all aspects of animal healthcare including medicine, supplements, diagnostics, medical devices.

Medicine, in turn, includes drugs, pharmaceutical agents, excipients, vitamins, minerals and nutritional supplements, .

Calcium compounds means compounds that comprise calcium, copper compounds means compounds that comprise copper, zinc compounds means compounds that comprise zinc.

The present invention relates to a cartridge suitable for use in veterinary applications, such as for the delivery of medicine and other substances to mammals, e.g. ruminants, working animals, pet animals, such as cats, dogs, rabbits, guinea pigs, hamsters, and sporting animals, in particular herbivores such as cattle, horses, goats sheep, lamas, alpacas and camels. In an embodiment the cartridge comprises a non-woven cellulose tube having a closed end and an open end, wherein at least one cross-sectional area of the closed end is axially symmetric in relation to the longitudinal axis of the tube. The closed end of the cartridge being axially symmetric in relation to the longitudinal axis of the tube allows easy passage of the cartridge into the oesophagus of an animal. This easy passage can be aided further by the coating agent.

In an embodiment the cartridge further comprises a coating agent.

In a further embodiment the closed end of the cartridge is rotationally symmetric, and optionally is annular.

It is also possible that the open end of the cartridge is annular or non-annular. In one embodiment the open end is annular.

Various means of coating the cartridge are possible. In one embodiment the coating agent is integrated into the cellulose or is coated on the cellulose. This provides for dry cartridges that can be easily stored before use.

There are several ways in which the open end of the cartridge can be closed. The cartridge can be closed to prevent its contents leaking out before the cartridge has released its delivery destination. In an embodiment the open end is plugged *e.g.* by folding or crushing the open end, or by inserting a cellulose cap, a disintegrating cap or a soluble cap.

In an embodiment the cartridge has an outer diameter in the range of 3 to 100 mm, a length in the range 5 to 400 mm and an internal volume in the range of about 0.15 ml to about 12500 ml. The size of the cartridge is selected based on the animal to which the cartridge is to be administered. The cartridge is typically made of cellulose in one embodiment. In a further embodiment the cellulose is tree-based cellulose. Tree - based cellulose can be sourced from hardwood or softwood.

In an embodiment the tree-based cellulose is sourced from trees selected from the group of pine, spruce, eucalyptus, oak ash, beech and combinations thereof. In one embodiment cellulose is sourced from modified wood fibres, pulp fibres and regenerated wood fibres. In further embodiments the cellulose is sourced from natural sources selected from the group of cotton, linen, reed canary grass, flax, jute, sisal, hemp, ramie, rice straw, sugar beet, banana, potato, bamboo and abaca, and combinations thereof. The cellulose can also be recycled cellulose. In an embodiment the cellulose is recycled from paper and/or board products.

When the cartridge is administered to the animal, the nonwoven cellulose cartridge is disintegrated to a pulp, *e.g.* in ruminants the cartridge disintegrates to pulp in the rumen liquid and is digested by rumen microbes.

The supplement or substance to be administered to the animal is filled into the cartridge. In an embodiment, the supplement or substance is released into the animal as the cartridge disintegrates. In ruminants, for example, disintegration is caused by wetting with rumen liquids and peristaltic movement in the rumen or by rumen microbes or by a combination thereof. The cartridge is broken down rapidly by wetting and then the fibres are digested by microbes found in the rumen.

The rate at which the cartridge disintegrates and delivers its contents can be varied by the thickness of the nonwoven cellulose. In an embodiment the nonwoven cellulose is less than or equal to 2.0 mm thick, preferably 0.5 to 1.9 mm thick, particularly 0.6 to 1.8 mm thick, suitably 0.7 to 1.6 mm thick, more preferably 0.8 to 1.5 mm thick, more particularly 0.9 to 1.4 mm thick most suitably 1.0, 1.1, 1.2 or 1.3 mm thick.

Rate of disintegration can also be dependent on degree of polymerization (DP) of the cellulose fibres, the DP in turn depending on the origin if the cellulose. Typically, cellulose fibres with a low DP, *e.g.* cellulose fibres having a DP in the range of 300 to 1000 units, can provide densely packed cellulose, which does not absorb water as readily as less densely packed cellulose and/or cellulose with a DP of 1001 or more and therefore would provide cartridges that disintegrate over a longer period of time.

For cartridges that should disintegrate quickly, nonwoven cellulose in which the cellulose fibres are packed less densely and/or longer cellulose fibres, e.g. with a degree of polymerization of 1001 units or more can be used. The density of packing of the fibres is varied during formation of the nonwoven cellulose. The density of packing is directly proportional to compression of the cellulose fibres during production of the nonwoven cellulose. In simple terms, rate of disintegration of the nonwoven cellulose cartridge is related to how readily the nonwoven cellulose cartridge absorbs water or bodily fluids, in other words how easily it swells. In turn, these two things depend on the structure of cellulose; how much of the structure is crystalline and how much is amorphic. Thus, the rate of disintegration of the nonwoven cellulose cartridge can be varied by producing the cartridge from cellulose fibres of increasing crystallinity to reduce the ability of the nonwoven cellulose cartridge to absorb water and disintegrate more slowly and vice versa, by producing the cartridge from cellulose fibres of decreasing crystallinity to increase the ability of the nonwoven cellulose cartridge to absorb water and disintegrate more rapidly.

As mentioned above, a coating agent can aid passage of the cartridge along the oesophagus. In an embodiment the coating agent is present in amount of 0.01 to 50 by wt-% of the cartridge.

Various coating agents can be applied to the cartridge. In an embodiment the coating agent is a lubricating agent selected from fatty acid stearates, such as magnesium stearate or zinc stearate, natural and synthetic waxes such as N,N' ethylenebisstearamide, polyvinyla alcohol (PVA), and carboxymethyl cellulose.

The cartridges of the present invention can be used in various applications. One embodiment provides a cartridge comprising a non-woven cellulose tube having a closed end and an open end, wherein at least one cross-sectional area of the closed end is axially symmetric in relation to the longitudinal axis of the tube for use in veterinary applications, such as medicine. A further embodiment provides a cartridge according to any of the above-described embodiments for use in veterinary applications, such as medicine.

A still further embodiment provides a cartridge for use in medicine further comprising one or more supplements selected from the group consisting of calcium compounds, copper compounds and zinc compounds.

A further embodiment provides a cartridge for oral delivery. The cartridge may contain solids, powders, gels, emulsions, solutions, or liquids.

In an embodiment a cartridge is provided for the delivery of a substance, such as a solid substance or a liquid substance.

In a further embodiment the cartridge can be used for the delivery of a substance, medical device, diagnostic device, magnet or magnets, or a combination thereof.

Further embodiments of the invention relate to a method of producing a cellulose cartridge. In an embodiment the method of producing the cartridge comprises the steps of moulding a feed grade or higher purity grade cellulose-water paste on a mould to produce a single homogenous cartridge, drying the cartridge.

In a further embodiment the dried cartridge is dipped in an aqueous coating solution to coat the cartridge in a coating, and drying the coated cartridge.

In one embodiment the method comprises a second dipping step and a further drying step after the second dipping step. The second dipping step provides a second layer of coating agent to the cartridge.

In the case of certain substances to be administered, the cartridge should be closed to prevent the substance leaking from the cartridge before the cartridge reaches the part of the animal's digestive system where the substance is to be delivered. In the case of ruminants this is the rumen.

In an embodiment the method comprises a further step of adding a substance, medical device or diagnostic device to the cartridge and plugging the cartridge e.g. by folding or crushing the open end, or by inserting a cellulose cap, a disintegrating cap or a soluble cap.

Further embodiments relate to a kit of parts. In one embodiment a kit is provided comprising a cartridge according to any of the above-described embodiment and a delivery applicator. In one embodiment the kit further comprises a lubricating agent and optionally a local anaesthetic.

Size, release time, release mechanism can be adjusted according to indication by selection of cartridge design, cellulose source, and used additives (CMC, kaolin). Design can be altered by diameter, length, wall thickness and by how the cartridge is plugged or stopped. Plugging or stoppering can vary by design (folding or separate stop solutions) or by material (cellulose, starch, wax, gelatine). Although most suitable for herbivores, such as cow, horse, goat, sheep, alpaca, lama, camel, the inventions can be used with other animals, too, since pure cellulose will travel through gastro-intestinal track and work as roughage. The inner or outer wall of cartridge can also be coated with a substance such as gelatine halting or slowing the penetration of liquids from rumen into the cartridge allowing delayed release of content, or from inside of cartridge into mouth or oesophagus allowing administration of liquids.

### EXAMPLES

### Example 1

A cartridge was produced by molding feed grade cellulose-water paste to produce single homogenous piece. Dried cartridge was dipped in CMC water solution of desired viscosity and dried to give cartridge a coating that allows easy passage of the cartridge through a subject's digestive system. A test cartridge with dimentions of 32 mm in outer diameter and of 150 mm in length was coated in 1.0 wt-% calculated from the total weight of the solution food grade CMC with viscosity in the range of 2500 to 3500 mPas and dried in fan assisted oven in 50 degrees of Celsius for 5 hours. Cartridge was filled with solid sodium chloride salt as test substance, folded closed and administered to cow with a freely available applicator. The arrival of cartridge into cow's rumen and the release of NaCl was observed in fistulated cows. The *in vivo* test showed that the cartridge arrived in the cows rumen in *ca.* 20 seconds from the application. Disintegration starts immediately and within minutes the cartridge is returned to soft pulp, partly hold together by the CMC coating. The release of content substance follows.

### Example 2

A cartridge according to Example 1 was filled with NaCl as a test substance, and folded closed, and placed in a water bath at 39 °C. The water bath was stirred once every 30 seconds. After 30 seconds, it was observed that the folded end opened and NaCl started to be released into the water bath. After 60 seconds, the cartridge began to swell and cellulose fibres started to flake from the cartridge.

It is to be understood that the embodiments of the invention disclosed are not limited to the particular structures, process steps, or materials disclosed herein, but are extended to equivalents thereof as would be recognized by those ordinarily skilled in the relevant arts. It should also be understood that terminology employed herein is used for the purpose of describing particular embodiments only and is not intended to be limiting.

Reference throughout this specification to one embodiment or an embodiment means that a particular feature, structure, or characteristic described in connection with the embodiment is included in at least one embodiment of the present invention. Thus, appearances of the phrases "in one embodiment" or "in an embodiment" in various places throughout this specification are not necessarily all referring to the same embodiment. Where reference is made to a numerical value using a term such as, for example, about or substantially, the exact numerical value is also disclosed.

As used herein, a plurality of items, structural elements, compositional elements, and/or materials may be presented in a common list for convenience. However, these lists should be construed as though each member of the list is individually identified as a separate and unique member. Thus, no individual member of such list should be construed as a de facto equivalent of any other member of the same list solely based on their presentation in a common group without indications to the contrary. In addition, various embodiments and example of the present invention may be referred to herein along with alternatives for the various components thereof. It is understood that such embodiments, examples, and alternatives are not to be construed as de facto equivalents of one another, but are to be considered as separate and autonomous representations of the present invention.

Furthermore, the described features, structures, or characteristics may be combined in any suitable manner in one or more embodiments. In the following description, numerous specific details are provided, such as examples of lengths, widths, shapes, etc., to provide a thorough understanding of embodiments of the invention. One skilled in the relevant art will recognize, however, that the invention can be practiced without one or more of the specific details, or with other methods, components, materials, etc. In other instances, well-known structures, materials, or operations are not shown or described in detail to avoid obscuring aspects of the invention.

While the forgoing examples are illustrative of the principles of the present invention in one or more particular applications, it will be apparent to those of ordinary skill in the art that numerous modifications in form, usage and details of implementation can be made without the exercise of inventive faculty, and without departing from the principles and concepts of the invention. Accordingly, it is not intended that the invention be limited, except as by the claims set forth below.

The verbs "to comprise" and "to include" are used in this document as open limitations that neither exclude nor require the existence of also un-recited features. The features recited in depending claims are mutually freely combinable unless otherwise explicitly stated. Furthermore, it is to be understood that the use of "a" or "an", that is, a singular form, throughout this document does not exclude a plurality.

### INDUSTRIAL APPLICABILITY

At least some embodiments of the present invention find industrial application in agriculture and veterinary applications, *e.g*. the delivery of supplements, diagnostic devices, medical devices, liquid substances and solid substances to animals, in particular mammals. Further applications are found in medicine.

### CITATION LIST

### Patent Literature

WO 2006/072252
US 5,395,622

## Claims

1. A cartridge for delivery of medicine to mammals in veterinary applications comprising a non-woven cellulose tube having a closed end and an open end, wherein at least one cross-sectional area of the closed end is axially symmetric in relation to the longitudinal axis of the tube and the open end is plugged *e.g.* by folding or crushing the open end, or by inserting a cellulose cap, a disintegrating cap or a soluble cap.

2. The cartridge according to claim 1 further comprising a coating agent.

3. The cartridge according to claim 1 or 2, wherein the closed end is rotationally symmetric, and optionally is annular.

4. The cartridge according to any of claims 1 to 3, wherein the open end is annular.

5. The cartridge according to any of the preceding claims, wherein the coating agent is integrated into the cellulose or is coated on the cellulose.

6. The cartridge according to any of the preceding claims, wherein the cartridge has an outer diameter in the range of 3 to 100 mm, a length in the range 5 to 400 mm and an internal volume in the range of about 0.15 ml to about 12500 ml.

7. The cartridge according to any of the preceding claims, wherein the coating agent is present in amount of 0.01 to 50 by wt-% of the cartridge.

8. The cartridge according to any of the preceding claims, wherein the coating agent is a lubricating agent selected from fatty acid stearates, such as magnesium stearate or zinc stearate, natural and synthetic waxes e.g. N,N'-ethylenebisstearamide and carboxymethyl cellulose.

9. A cartridge according to any of claims 1 to 8 for use in veterinary applications.

10. A cartridge according to claim 9 for use in medicine.

11. The cartridge according to claim 9 or 10 further comprising one or more supplements selected from the group consisting of calcium compounds, copper compounds, and zinc compounds

12. The cartridge according to any of claims 1 to 11 for oral delivery.

13. The cartridge according to any of claims 1 to 12 for the delivery of a substance, such as a liquid substance or a solid substance, medical device, a diagnostic device, magnet or magnets or a combination thereof.

14. A method of producing a cellulose cartridge according to any of claims 1 to 13 for the delivery of medicine to mammals comprising
- moulding a feed grade cellulose-water paste on or in a mould to produce a single homogenous cartridge, and
- drying the cartridge.

15. The method according to claim 14 further comprising
- dipping the dried cartridge in an aqueous coating solution to coat the cartridge in a coating, and
- drying the coated cartridge.

16. The method according to claim 15, comprising a second dipping step and a further drying step after the second dipping step.

17. The method according to any of claims 14 to 16, comprising a further step of adding a substance, medical device, diagnostic device, magnet or magnets, or a combination thereof to the cartridge and plugging the cartridge e.g. by folding or crushing the open end, or by inserting a cellulose cap, a disintegrating cap or a soluble cap.

18. A kit comprising a cartridge according to any of claims 1 to 12 and a delivery applicator/gun.

19. Use of a cartridge according to any of claims 1 to 13 in veterinary applications such as the delivery of a substance or substances to mammals.
